(19) Europäisches Patentamt
European Patent Office
Office européen des brevets

(11) Veröffentlichungsnummer: **0 252 004**
**A1**

(12) # EUROPÄISCHE PATENTANMELDUNG

(21) Anmeldenummer: **87810348.0**

(22) Anmeldetag: **22.06.87**

(51) Int. Cl.$^4$: **A 61 K 37/54**
A 61 K 47/00

(30) Priorität: **26.06.86 CH 2583/86**

(43) Veröffentlichungstag der Anmeldung:
**07.01.88 Patentblatt 88/01**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Geller, Leo, Dr.**
**Rudolf Wackernagel-Strasse 14**
**CH-4125 Riehen (CH)**

**Glanzmann, Peter**
**J.J. Balmerstrasse 7**
**CH-4053 Basel (CH)**

**Küenzi, Martin, Dr.**
**Hüslimattstrasse 48**
**CH-4132 Muttenz (CH)**

**Weiss, Kurt**
**Im Brüel 12**
**CH-4312 Magden (CH)**

Patentansprüche für folgende Vertragsstaat: AT.

(54) **Pharmazeutische Zusammensetzungen für die parenterale Applikation.**

(57) Die vorliegende Erfindung betrifft pharmazeutische Zusammensetzungen für die parenterale Applikation von hochmolekularen Proteinen mit fibrinolytischer Aktivität, insbesondere Tissue Plasminogen Activator (TPA). Die pharmazeutischen Zusammensetzungen bilden in wässriger Phase Dispersionen von Mischmizellen, welche den Wirkstoff, z.B. TPA, verkapseln. Mizellbildner sind Phospholipide wie Lecithin und Gallensäurederivate. Die pharmazeutischen Zusammensetzungen werden zur Prophylaxe und Therapie von Thrombosen verwendet.

EP 0 252 004 A1

**Beschreibung**

Pharmazeutische Zusammensetzungen für die parenterale Applikation

Die vorliegende Erfindung betrifft pharmazeutische Zusammensetzungen für die parenterale Applikation von Proteinen mit fibrinolytischer Aktivität, insbesondere Gewebe-Plasminogen-Aktivator (TPA), und die Verwendung dieser pharmazeutischen Zusammensetzungen zur Prophylaxe oder Therapie beispielsweise von Thrombosen.

Es ist bekannt, dass hochmolekulare Fibrinolytika wie Streptokinase oder Urokinase nur geringe Fibrinspezifität besitzen, was zu hohen Dosierungen zwingt, wobei Nebenwirkungen wie allergische Reaktionen, anaphylaktische Schocks, Hämolyse, Blutungen, Pyrogenwirkung etc. auftreten können. Das in verschiedenen Publikationen, z.B. The New England Journal of Medicine, 310, 10, 609-613 (1984) and 312, 14, 932-936, beschriebene hochmolekulare Fibrinolytikum Tissue Plasminogen Activator (TPA = Gewebe-Plasminogen-Aktivator) besitzt zwar eine verbesserte Spezifität für Fibrin, ist aber als gereinigter Feststoff, z.B. als Lyophilisat, nur schwer wasserlöslich, was zur Verabreichung von grossen Volumina Infusionslösung zwingt. Aufgrund der schlechten Wasserlöslichkeit ist TPA insbesondere für konzentrierte parenterale Lösungen, wie sie für Bolusapplikationen erforderlich sind, ungeeignet.

Der vorliegenden Erfindung liegt die Aufgabe zugrunde, durch Wahl geeigneter Trägermaterialien die Löslichkeit und Stabilität von Fibrinolytika in wässriger Phase zu erhöhen.

Die vorliegende Erfindung betrifft pharmazeutische Zusammensetzungen enthaltend

a) ein Phospholipid der Formel

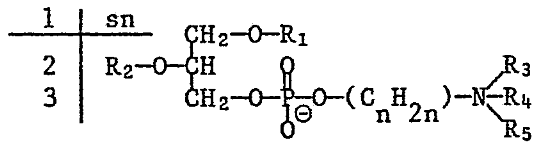

(I),

worin n zwei, drei oder vier ist, $R_1$ und $R_2$ unabhängig voneinander Alkyl, Alkenyl oder Acyl mit je 10-20 C-Atomen und $R_3$, $R_4$ und $R_5$ Wasserstoff oder $C_1$-$C_4$-Alkyl bedeuten,

b) ein Gallensäurederivat der Formel

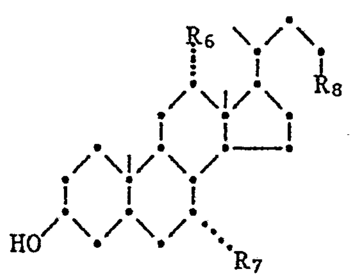

(II),

worin $R_6$ und $R_7$ unabhängig voneinander Wasserstoff, Hydroxy oder Oxo und $R_8$ Carboxy, verestertes oder amidiertes Carboxy bedeuten, und welches im Steroidgerüst als zusätzliche funktionelle Gruppen ein oder zwei Doppelbindungen enthalten kann,

c) ein hochmolekulares Protein mit fibrinolytischer Aktivität (Fibrinolytikum) und gegebenenfalls eine pharmazeutisch annehmbare Trägerflüssigkeit und/oder für die pharmazeutische Formulierung verwendbare Hilfsstoffe.

Die weiter vorn und im folgenden genannten allgemeinen Begriffe haben im Rahmen der Beschreibung der vorliegenden Erdingung vorzugsweise die folgenden Bedeutungen:

Der im Zusammenhang mit organischen Resten, z.B. Niederalkyl, Niederalkylen, Niederalkoxy, Niederalkanoyl etc., verwendete Ausdruck "Nieder" bedeutet, dass solche organischen Reste, falls nicht ausdrücklich anders definiert, bis einschliesslich 7 und beforzugt bis einschliesslich 4 Kohlenstoffatome enthalten.

Die Nomenklatur und Konfigurationsbezeichnung der Phospholipide der Formeln I und II erfolgt anhand der in Eur. J. of Biochem. 79, 11-21 (1977) "Nomenclature of Lipids" von der IUPAC-IUB Commission on Biochemical Nomenclature (CBN) gegebenen Empfehlungen (sn-Nomenklatur, stereospecific numbering).

In einem Phospholipid der Formel I (Komponente a)) ist Alkyl $R_1$ und $R_2$ vorzugsweise geradkettig mit einer geraden Anzahl von 10-20 C-Atomen, z.B. n-Decyl, n-Dodecyl, n-Tetradecyl, n-Hexadecyl oder n-Octadecyl.

Alkenyl $R_1$ und $R_2$ ist vorzugsweise geradkettig mit einer geraden Anzahl von 10-20 C-Atomen und einer Doppelbindung, z.B. 6-cis-, 6-trans-, 9-cis- oder 9-trans-dodecenyl, -tetradecenyl, -hexadecenyl, -octadecenyl oder -icosenyl, insbesondere 9-cis-octadecenyl.

Acyl $R_1$ und $R_2$ ist vorzugsweise geradkettig mit einer geraden Anzahl von 10-20 C-Atomen, z.B.

$C_{10}$-$C_{20}$-Alkanoyl oder $C_{10}$-$C_{20}$-Alkenoyl mit einer Doppelbindung.

$C_{10}$-$C_{20}$-Alkanoyl $R_1$ und $R_2$ ist beispielsweise n-Dodecanoyl, n-Tetradecanoyl, n-Hexadecanoyl oder n-Octadecanoyl.

$C_{10}$-$C_{20}$-Alkenoyl $R_1$ und $R_2$ ist beispielsweise 6-cis-, 6-trans-, 9-cis- oder 9-trans-dodecenoyl, -tetradecenoyl, -hexadecenoyl, -octadecenoyl oder -icosenoyl, insbesondere 9-cis-octadecenoyl (oleoyl).

Alkyl $R_3$, $R_4$ und $R_5$ ist vorzugsweise Methyl.

Die pharmazeutischen Zusammensetzungen können Gemische von natürlich vorkommenden Phospholipiden der Formel I pflanzlichen oder tierischen Ursprungs oder synthetische, im wesentlichen reine Phospholipide der Formel I enthalten.

Bevorzugt sind natürlich vorkommende Kephaline der Formel I, worin $R_3$, $R_4$ und $R_5$ Wasserstoff bedeuten, oder natürlich vorkommende Lecithine, worin $R_3$, $R_4$ und $R_5$ Methyl bedeuten, z.B. Kephalin oder Lecithin aus Sojabohnen, Rinderhirn, Rinderleber oder Hühnerei mit verschiedenen oder identischen $C_{10}$-$C_{20}$-Alkanoyl- oder $C_{10}$-$C_{20}$-Alkenoylgruppen, synthetisches Kephalin oder Lecithin mit identischen $C_{10}$-$C_{20}$-Alkanoyl- oder $C_{10}$-$C_{20}$-Alkenoylgruppen, oder synthetisches Kephalin oder Lecithin, worin $R_1$ $C_{10}$-$C_{20}$-Alkanoyl, z.B. n-Dodecanoyl, n-Tetradecanoyl, n-Hexadecanoyl oder n-Octadecanoyl, und $R_2$ $C_{10}$-$C_{20}$-Alkenoyl, z.B. 6-cis-, 6-trans-, 9-cis-oder 9-trans-octadecenoyl bedeuten, insbesondere 1-n-Hexadecanoyl-2-(9-cis-octadecenoyl)-3-sn-phosphatidylcholin.

In einem Gallensäurederivat (Cholsäurederivat) der Formel II stellen die punktierten Linien eine Einfachbindung dar, wenn $R_6$ und/oder $R_7$ Wasserstoff oder Hydroxy bedeuten oder stellen eine Doppelbindung dar, wenn $R_6$ und/oder $R_7$ Oxo bedeuten.

Verestertes Carboxy $R_8$ ist z.B. $C_1$-$C_4$-Alkoxycarbonyl, z.B. Methoxycarbonyl.

Amidiertes Carboxy $R_8$ ist z.B. $C_1$-$C_4$-Alkylaminocarbonyl, z.B. Dimethylaminocarbonyl, insbesondere $C_2$-$C_4$-Alkylaminocarbonyl substituiert durch Carboxy oder Sulfo, z.B. 2-Carboxy- oder 2-Sulfoethylaminocarbonyl.

Die Verbindungen der Formel II können im Steroidgerüst als zusätzliche funktionelle Gruppe ein oder zwei Doppelbindungen enthalten, z.B. in 7-, 9- oder in 11-Stellung.

Bevorzugt sind Gallensäurederivate der Formel II, worin $R_8$ Carboxy, Sulfo oder 2-Carboxy- oder 2-Sulfoethylaminocarbonyl ist, z.B. Cholsäure, Deoxycholsäure, Glycodeoxycholsäure, Taurodeoxycholsäure, Chenodeoxycholsäure, Glycochenodeoxycholsäure, Taurochenodeoxycholsäure, Glycocholsäure oder Taurocholsäure.

Besonders bevorzugt sind Gallensäurederivate der Formel II, worin $R_6$ und $R_7$ Hydroxy und $R_8$ Carboxy oder 2-Carboxy- oder 2-Sulfoethylaminocarbonyl bedeuten, z.B. Cholsäure, Glycocholsäure oder Taurocholsäure.

Hochmolekulare Proteine mit fibrinolytischer Aktivität (Fibrinolytika) sind beispielsweise Enzyme mit einer Molmasse grösser als 10000, insbesondere grösser als 30000 und der allgemeinen Bezeichnung Plasminogenaktivatoren, welche die Umwandlung von Plasminogen, einer inaktiven und zum proteolytischen System gehörenden, im Blut enthaltenen Proenzymform von Plasmin in das proteolytisch wirksame Enzym Plasmin katalysieren. Plasmin seinerseits bewirkt u.a. die Proteolyse des Fibrins zu Fibrinopeptiden, die ihrerseits über Thrombinhemmung und Störung der Polymerisation des Fibrins gerinnungshemmend wirken.

Plasminogenaktivatoren sind u.a. die in der thrombolytischen Therapie verwendbaren ein- oder zweikettigen Serinproteasen, Urokinase und Gewebe-Plasminogen-Aktivator (TPA) sowie Streptokinase.

Bevorzugt ist einkettige Urokinase, welche aus menschlichem Urin oder dem Kulturfiltrat von Nierenzellkulturen oder genetisch rekombinierten Mikroorganismen oder Säugerzellen erhältlich ist, TPA aus Zellkulturen von gesundem oder tumorösem Zellgewebe von Säugetieren oder Menschen, TPA exprimiert in Kulturen von genetisch rekombinierten Mikroorganismen, z.B. Eukaryoten wie Pilzen, z.B Aspergillusarten, oder Hefen, oder Prokaryoten, z.B. Escherichia Coli, oder genetisch rekombinierten Säugerzellen, Mutanten oder Hybridformen von Urokinase und TPA, Konjugate von TPA, z.B. mit Proteinen, sowie denaturiertes TPA mit fibrinolytischer Wirkung und durch Ersatz von Aminosäurefragmenten bzw. Derivatisierung am C-und/oder N-Terminus chemisch modifiziertes TPA.

Die Herstellung von Urokinase bzw. ihre Isolierung und Reinigung ist in zahlreichen Publikationen beschrieben, z.B. direkt durch DNA-Rekombination gemäss Europäische Patentanmeldung (EP-A) 92 182 oder U.S. Patentschrift (USP) 4,370,417 oder Bildung einer pro-Form gemäss EP-A-0 154 272 oder EP-A-0 210 279, die in fibrinolytisch wirksame Urokinase überführbar ist.

Die Herstellung von TPA bzw. die Isolierung und Reinigung davon ist in zahlreichen Publikationen beschrieben, z.B. die Herstellung aus Kulturfiltraten von humanen Embryo-Nierenzellkulturen, siehe USP 4,505,893, humanen Embryo-Lungenzellkulturen, siehe EP-A-151,996, aus Kulturfiltraten von humanen Zellinien von Melanoma-zellen des Typs Bowes, siehe EP-A-113,319, aus Hefezellkulturen gemäss EP-A-143,081, oder aus Kulturen von genetisch manipulierten Mikroorganismen, siehe EP-A-93,619.

Die Herstellung von Mutanten von TPA ist in der Deutschen Offenlegungsschrift (DE-A) 3 613 390 beschrieben, von denaturiertem TPA in EP-A-0 196 920, von chemisch modifiziertem TPA in EP-A-207 589 und WO 86/01538 sowie von Hybridformen von TPA und Urokinase in EP-A-155 387.

In einer pharmazeutisch annehmbaren Trägerflüssigkeit sind die weiter vorn genannten Komponenten a), b) und c) sowie gegebenenfalls pharmazeutisch annehmbare feste Trägermaterialien dispergiert.

Für die parenterale Applikationsform der erfindungsgemässen pharmazeutischen Zusammensetzungen ist die Trägerflüssigkeit isotonisch neutral (pH 7.2-7.4). Diese Bedingungen lassen sich in üblicher Weise durch

Zusatz von Kochsalz, Glucose etc. herstellen.

Die Komponenten a) - Phospholipide - und b) - Gallensäurederivate -bilden vorzugsweise in der genannten Trägerflüssigkeit Mischmizellen, welche die Komponente c) - Protein - einschliessen bzw. verkapseln. Die Herstellung der Mischmizellen enthaltenden wässrigen Phase kann durch Vermischen der Komponenten und deren Dispersion in der wässrigen Phase erfolgen.

Mit den für pharmazeutische Formulierung verwendbaren Hilfsstoffen kann die Mischmizellen enthaltende Trägerflüssigkeit zur Erhöhung der Stabilität der parenteralen Applikationsform vermischt sein. Mit solchen Hilfsstoffen kann sowohl die homogene Mischung, z.B. das Lyophilisat, der Komponenten a), b) und c) als auch das Lyophilisat der Mischmizellen enthaltenden Trägerflüssigkeit versetzt sein.

Geeignete Hilfsstoffe sind beispielsweise für die pharmazeutische Formulierung verwendbare grenzflächenaktive Stoffe, sog. Tenside, insbesondere nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern wie Polyoxyethylen-sorbitanmonolaurat, -oleat, -stearat, -palmitat, -tristearat oder -trioleat, Polyethylenglycol-Fettsäureester wie Polyoxyethylstearat, Polyethylenglycol-400-stearat, Polyethylenglycol-2000-stearat, insbesondere Ethylenoxid-Propylenoxid Blockpolymere vom Typ Pluronics® oder Synperonic® (ICI).

Zusätzlich zu diesen grenzflächenaktiven Stoffen, welche als Löslichkeitsvermittler fungieren, können die erfindungsgemässen pharmazeutischen Zusammensetzungen gegebenenfalls feste oder flüssige Trägermaterialien wie Kohlehydrate enthalten, z.B. Lactose, Saccharose, Mannit oder Sorbit, Niederalkanole, z.B. Ethanol oder Isopropanol oder Mischungen davon, mehrwertige Alkohole, wie Glycerin, Ethylen- oder Propylenglycol, insbesondere Polynieder alkylenglycole, z.B. Polyethylen- oder Polypropylenglycol mit einer Kettenlänge von etwa 200 bis etwa 5000, vorzugsweise von etwa 300 bis etwa 1500, Gliedern.

Zur Verhinderung von Oxydationsreaktionen des Wirkstoffs und der Trägermaterialien, insbesondere der Phospholipide, können Anti-oxidanzien, wie Natriumascorbat, Natriumhydrogensulfit, Natriumpyrosulfit, Natriumsulfit, Ascorbenpalmitat oder Tocopherole zugesetzt werden.

Die pharmazeutischen Zusammensetzungen der vorliegenden Erfindung besitzen wertvolle pharmakologische Eigenschaften. Aufgrund ihrer fibrinolytischen Aktivität können sie prophylaktisch oder therapeutisch zur Behandlung von Thrombosen oder durch Thrombosen verursachten Krankheiten wie Arteriosklerosis, Myokardinfarkt, Apoplexie, Venenthrombosen, Thromboembolien, Thrombophlebitis, etc. verwendet werden.

Proteine mit fibrinolytischer Aktivität in Form von Mischmizellen lassen sich ohne die bekannten Nachteile, z.B. Ueberdosierung und Nebenwirkungen, Adsorptionsphänomene an Ampullenwandungen aufgrund schlechter Löslichkeit, ungenügender Stabilität etc., in definierter Konzentration und Menge herstellen, lagern und applizieren.

So lassen sich beispielsweise die in wässriger Phase schlecht wasserlöslichen Fibrinolytika wie Streptokinase, Urokinase oder TPA in einer für den jeweiligen Zustand und Bedarf des Patienten dosierten Mindestmenge und Konzentration, die zur Erzielung eines therapeutischen Effekts notwendig ist, applizieren und so die Belastungen durch hohe Dosierungen vermeiden, wie sie bei der Anwendung von Streptokinase und Urokinase bekannt sind. Die erfindungsgemässen pharmazeutischen Zusammensetzungen ermöglichen die Herstellung einer parenteralen Darreichungsform für Fibrinolytika, welche ausserhalb des Klinikbereichs einsetzbar ist. So lassen sich Bolusapplikationen herstellen, welche die parenterale Zufuhr konzentrierter Lösungen, insbesondere für den notfallmässigen Bedarf, ermöglichen. Solche Bolusapplikationsformen lassen sich auch mit der in der Klinik erfolgenden intravenösen Zufuhr kombinieren, indem man nach einem Notfall, z.B. nach einer Thrombose, eine einmalige Bolusapplikation durchführt und nach Verabreichung dieser initialen Stossdosis in der Klinik die übliche intravenöse Behandlung mit grösseren Volumina und niedrigeren Konzentrationen nach erfolgter Stabilisierung des Gesundheitszustands fortsetzt.

Geeignete Volusapplikationsformen für die einmalige Verabreichung enthalten beispielsweise 5 - 50 mg TPA in ca. 1 - 5 ml Wasser. Die geeignete Tagesdosis von TPA beträgt ca. 10-200 mg, insbesondere 20-100 mg, wobei Infusionslösungen enthaltend 20, 40 oder 60 mg TPA jeweils 1-2 Stunden appliziert werden können. Es lassen sich auch Infusionslösungen mit unterschiedlicher Konzentration verabreichen, z.B. eine Stunde lang eine Infusionslösung enthaltend 40 mg TPA und anschliessend jeweils eine Stunde lang zwei Infusionslösungen enthaltend 20 mg TPA oder umgekehrt.

Die erfindungsgemässen pharmazeutischen Zusammensetzungen eignen sich daher zur Herstellung von Injektions- oder Infusionslösungen, indem man diese vor ihrer Anwendung aus Trockenpräparaten enthaltend die Komponenten a), b) und c) durch Versetzen mit Trägerflüssigkeit, z.B. isotonischer Kochsalz- oder Glucoselösung, herstellt. Die Infusionslösungen werden nach Bedarf sterilfiltriert, z.B. über Filter mit kleinem Porendurchmesser, z.B. 0,45 Mikrometer oder kleinerem Durchmesser.

Die pharmazeutischen Zusammensetzungen können auch in Dosiseinheitsform verwendet werden, die ca. 5-100 mg der aktiven Komponente a), z.B. TPA, enthalten. Je nach Art der Erkrankung des Patienten, seinem Alter und Zustand, können unterschiedliche oder gleiche Dosismengen ein- bis mehrmals am Tag verabreicht werden, die ca. 5 bis 200 mg Wirkstoff aufweisen.

Die vorliegende Erfindung betrifft bevorzugt pharmazeutische Zusammensetzungen enthaltend

a) ein synthetisches oder natürliches Phospholipid der Formel I, worin n zwei ist, $R_1$ und $R_2$ Acyl mit je 10-20 C-Atomen und $R_3$, $R_4$ und $R_5$ Methyl bedeuten,

b) ein Gallensäurederivat der Formel II, worin $R_6$ Hydroxy, $R_7$ Wasserstoff oder Hydroxy und $R_8$ Carboxy, Sulfo oder 2-Carboxy- oder 2-Sulfoethylaminocarbonyl bedeuten,

c) ein hochmolekulares Protein mit fibrinolytischer Aktivität aus der Gruppe Streptokinase, Urokinase oder TPA und gegebenenfalls eine pharmazeutisch annehmbare Trägerflüssigkeit und/oder für die pharmazeutische Formulierung verwendbare Hilfsstoffe.

Die vorliegende Erfindung betrifft in erster Linie pharmazeutische Zusammensetzungen enthaltend

a) ein synthetisches oder natürliches Phospholipid der Formel I, worin n zwei ist, $R_1$ und $R_2$ verschiedene oder identische $C_{10}$ -$C_{20}$ -Alkanoyl- oder $C_{10}$ -$C_{20}$ -Alkenoylgruppen, z.B. n-Dodecanoyl, n-Tetradecanoyl, n-Hexadecanoyl oder n-Octadecanoyl, 9-cis-Dodecenoyl, -Tetradecenoyl oder -Hexadecenoyl oder 6-cis-, 6-trans-, 9-cis-, 9-trans- oder 11-cis-Octadecenoyl und $R_3$ , $R_4$ und $R_5$ Methyl bedeuten,

b) ein Gallensäurederivat der Formel II, worin $R_6$ und $R_7$ Hydroxy und $R_8$ 2-Carboxy- oder 2-Sulfoethylaminocarbonyl bedeuten,

c) einkettige Urokinase, welche aus menschlichem Urin oder dem Kulturfiltrat von Nierenzellkulturen oder genetisch rekombinierten Mikroorganismen oder Säugerzellen erhältlich ist, TPA aus Zellkulturen von gesundem oder tumorösem Zellgewebe von Säugetieren oder Menschen, TPA exprimiert in Kulturen von genetisch rekombinierten Mikroorganismen, z.B. Eukaryoten wie Pilzen, z.B. Aspergillusarten, oder Hefen, oder Prokaryoten, z.B. Escherichia Coli, oder genetisch rekombinierten Säugerzellen, Mutanten und Hybridformen von Urokinase und TPA, Konjugate von TPA, z.B. mit Proteinen, sowie denaturiertes TPA mit fibrinolytischer Wirkung oder durch Ersatz von Aminosäurefragmenten bzw. Derivatisierung am C- und/oder N-Terminus chemisch modifiziertes TPA und gegebenenfalls eine pharmazeutisch annehmbare Trägerflüssigkeit und/oder für die pharmazeutische Formulierung verwendbare Hilfsstoffe.

Die vorliegende Erfindung betrifft vor allem pharmazeutische Zusammensetzungen enthaltend

a) ein Phospholipid der Formel I natürlicher Herkunft aus Pflanzen oder Tieren, worin $R_1$ und $R_2$ verschiedene $C_{10}$ -$C_{20}$ -Alkanoyl- oder $C_{10}$ -$C_{20}$ -Alkenoylgruppen, z.B. n-Dodecanoyl, n-Tetradecanoyl, n-Hexadecanoyl oder n-Octadecanoyl, 9-cis-Dodecenoyl, -Tetradecenoyl oder -Hexadecenoyl oder 6-cis-, 6-trans-, 9-cis-, 9-trans- oder 11-cis-Octadecenoyl und $R_3$ , $R_4$ und $R_5$ Methyl darstellen,

b) ein Gallensäurederivat der Formel II, worin $R_6$ und $R_7$ Hydroxy und $R_8$ 2-Carboxy- oder 2-Sulfoethylaminocarbonyl bedeuten,

c) einkettige Urokinase, welche aus menschlichem Urin oder dem Kulturfiltrat von Nierenzellkulturen oder genetisch rekombinierten Mikroorganismen oder Säugerzellen erhältlich ist, TPA aus Zellkulturen von gesundem oder tumorösem Zellgewebe von Säugetieren oder Menschen, TPA exprimiert in Kulturen von genetisch rekombinierten Mikroorganismen, z.B. Eukaryoten wie Pilzen, z.B. Aspergillusarten, oder Hefen, oder Prokaryoten, z.B. Escherichia Coli, und von genetisch rekombinierten Säugerzellen, oder Hybridformen von Urokinase und TPA und gegebenenfalls eine pharmazeutisch annehmbare Trägerflüssigkeit und/oder für die pharmazeutische Formulierung verwendbare Hilfsstoffe.

Die pharmazeutischen Zusammensetzungen der vorliegenden Erfindung, welche in Form von Mischmizellen in wässriger Phase anwendbar sind, lassen sich durch Vermischen der Komponenten a), b) und c) und gegebenenfalls der Hilfsstoffe und deren Dispersion in wässriger Phase oder durch Bildung eines Films oder Lyophilisats aus den genannten Komponenten und anschliessende Dispersion und, wenn notwendig, Abpufferung der wässrigen Phase auf pH 7.2-7.4 und gewünschtenfalls Anreicherung und/oder Abtrennung der erhältlichen Mischmizellen herstellen.

Man dispergiert beispielsweise durch Schütteln, z.B. mit einem Vortex-Mischer, oder Rühren der wässrigen Phase, der man gleichzeitig oder nacheinander die Komponenten a), b) und die Einschlusskomponente c) zugesetzt hat. Die Zugabe der Hilfsstoffe kann gleichzeitig mit den genannten Komponenten vor oder nach der Dispersion erfolgen. Die Bildung von Mischmizellen wird in der Regel durch leichtes Erwärmen, z.B. bei Temperaturen von Raumtemperatur bis 80°C durchgeführt.

Die Filmbildung erfolgt durch Auflösen der Komponenten a), b) und c) in einem organischen Lösungsmittel und Abdampfen dieses Lösungsmittels.

Für die Filmbildung geeignete Lösungsmittel sind beispielsweise unsubstituierte oder substituierte, z.B. halogenierte, aliphatische oder cycloaliphatische Kohlenwasserstoffe, z.B. n-Hexan, Cyclohexan, Methylenchlorid oder Chloroform, Alkohole, z.B. Methanol oder Ethanol, Carbonsäureester oder -Amide, z.B. Essigsäureethylester oder Dimethylformamid, oder Ether, z.B. Diethylether, Tetrahydrofuran oder Dioxan, oder Mischungen dieser Lösungsmittel.

Das Lösungsmittel wird vorzugsweise im Vakuum, gegebenenfalls im Hochvakuum, oder durch Abblasen mit Schutzgas, z.B. trockenem Stickstoff, entfernt.

Das Lyophilisat wird z.B. durch Auflösen der Komponenten a), b) und c) in solchen organischen Lösungsmitteln, die mit diesen Komponenten bei der Temperatur des Lyophilisierungsvorgangs einen Festkörper bilden und einen Schmelzpunkt höher als 0° besitzen, z.B. Eisessig oder Dioxan, insbesondere tert-Butanol, hergestellt.

Statt des Films oder Lyophilisats lässt sich eine homogene Mischung ebenfalls in Form eines Pulvers durch Sprühtrocknen einer Lösung der Komponenten a), b) und c) in einem organischen Lösungsmittel mit niedrigem Schmelzpunkt, z.B. Chloroform, herstellen.

In der wässrigen Phase oder in der homogenen Mischung beträgt das Molverhältnis der Komponente a) zur Komponente b) ca. 1:0,1 bis 1:2. Bevorzugt sind die Mischungsverhältnisse von ca. 1:0,1 bis 1:0,9 und 1:1,5 bis 1:2. Besonders bevorzugt ist das Mischungsverhältnis 1:0,8 bis 1:1,5.

Der Wirkstoffanteil (Komponente c)) in der wässrigen Phase kann über weite Grenzen variieren und ca. 0,1

mg bis 100 mg/ml betragen.

Der Anteil der Komponenten a) und b) allein kann ebenfalls über weite Grenzen variieren und z.B. 10-300 mg/ml wässrige Phase betragen.

Die erfindungsgemäss herstellbaren wässrigen Dispersionen neutralisiert oder puffert man auf pH 5,8-7,8, vorzugsweise pH 7,2-7,4, ab. Dies kann durch Zugabe einer physiologisch annehmbaren Säure oder Base oder einer Pufferlösung erfolgen. Physiologisch annehmbare Säuren sind z.B. verdünnte wässrige Mineralsäuren, z.B. verdünnte Salzsäure, Schwefelsäure oder Phosphorsäure, oder organische Säuren wie Essigsäure. Physiologisch annehmbare Basen sind insbesondere verdünnte wässrige Natriumhydroxid- oder Kaliumhydroxid-Lösungen. Man kann auch die genannten Komponenten in einer bereits auf physiologische pH-Werte abgepufferten wässrigen Lösung dispergieren, die man beispielsweise nach den in Hagers Handbuch der Pharmazeutischen Praxis, Springer Verlag Heidelberg, oder Remington's Handbook of Pharmaceutical Sciences für die Herstellung von Tris-Puffer oder Phosphatpuffer gegebenen Vorschriften erhält.

Bei Licht- oder Sauerstoffempfindlichkeit der erfindungsgemässen pharmazeutischen Zusammensetzungen führt man deren Herstellung unter Ausschluss von Licht und in Inertgasatmosphäre, z.B. unter Stickstoff oder Argon, durch. Zur Unterdrückung von unerwünschten Oxydationsreaktionen, insbesondere der Komponente a), kann man noch physiologisch geeignete Antioxydanzien wie Natriumascorbat der wässrigen Phase zusetzen.

Falls erwünscht lassen sich Mischmizellen-haltige wässrige Dispersionen mit Hilfe konventioneller Lyophilisierungsverfahren in Trockenpräparate überführen. Diese Trockenpräparate sind lagerstabil und können z.B. vor der Applikation in physiologischer Kochsalz- oder Glucoselösung sowie in den weiter vorn genannten Pufferlösungen suspendiert werden.

Die folgenden Beispiele illustrieren die Erfindung ohne diese zu beschränken.

Beispiel 1: Trockenpräparate enthaltend TPA

507,90 mg Sojalecithin (Sojaphosphatid NC 95, Fa. Nattermann, 90-96%-ig, Fettsäurezusammensetzung: Linolsäure 61-71 %, Oelsäure 6-13 %, Linolensäure 4-7 %, Palmitinsäure 10-15 %, Stearinsäure 1,5-3,5 %) und 278,10 mg Natriumglycocholat (reinst) werden in 2,15 g sterilem Wasser bei 80°C gelöst. Die lösung wird bei Raumtemperatur mit 1 N wässriger Natriumhydroxidlösung auf pH 7.4 gebracht. Danach werden 90,00 mg Mannit (reinst) und 45,00 mg TPA-Wirkstoff (lyophilisiert) hinzugegeben. Man rührt, bis die Lösung klar wird und ergänzt mit 2,35 g sterilem Wasser. Die Lösung wird sterilfiltriert, in Vialen abgefüllt und lyophilisiert.

Das den Wirkstoff TPA enthaltende Lyophilisat kann wie folgt hergestellt werden:

a) TPA aus Hefezellkulturen:

Das nach Beispiel 21b) bzw. 21c) der Europäischen Patentanmeldung Nr. 143 081 (Publikationsdatum 29.5.1985) erhaltene Eluat (8 ml) wird mit einer 0,1 M wässrigen Ammoniumacetatlösung (pH 5,0) auf das zehnfache verdünnt und mit einer Fliessgeschwindigkeit von ca. 25 ml pro Stunde bei Raumtemperatur auf eine mit 5 ml CM-Sepharose Fast Flow® (Pharmacia) gefüllte Säule, welche mit 0,1 M wässriger Ammoniumacetatlösung (pH 5,0) voräquilibriert wurde, gepumpt. Das Perkolat wird verworfen. Die Säule wird anschliessend mit drei Bettvolumen (BV), ca. 15 ml, 0,1 M wässriger Ammoniumacetatlösung (pH 5,0) und 2 Bettvolumen 0,1 M wässriger Ammoniumacetatlösung (pH 7,0) gewaschen. Die adsorbierten Proteine werden dann mit 1 M wässriger Ammoniumhydrogencarbonatlösung (pH 8,6) bei Raumtemperatur und einer Fliessgeschwindigkeit von ca. 5 ml pro Stunde bei ca. 1,0-1,5 bar Ueberdruck (Stickstoff) eluiert. Die Proteinkonzentration wird bei 280 nm mit einem UV-Monitor im Durchfluss gemessen. Ueber 85 % der adsorbierten "Aktivität" sind im Eluat enthalten und als scharfer Peak im UV-Monitor erkennbar. Nach Gefriertrocknung des Eluates erhält man ein amorphes, weisses Pulver, welches frei von Detergenzien ist.

b) TPA aus Bowes-Melanomazellkulturen

Das nach Beispiel 5b) bzw. 5c) der Europäischen Patentanmeldung Nr. 113 319 (Publikationsdatum 11.7.1984) erhaltene Eluat wird analog Beispiel 1a) aufgearbeitet und lyophilisiert. TPA lässt sich ebenfalls analog der von D. Collen et al. in Thromb. Haemostas 48 (3) 294-296 (1982) beschriebenen Methode gewinnen.

Beispiel 2: Trockenpräparate enthaltend TPA

406,50 mg Sojalecithin (Sojaphosphatid NC 95, Fa. Nattermann, 90-96%-ig, Fettsäurezusammensetzung: Linolsäure 61-71 %, Oelsäure 6-13 %, Linolensäure 4-7 %, Palmitinsäure 10-15 %, Stearinsäure 1,5-3,5 %) und 278,10 mg Natriumglycocholat (reinst) werden in 2,27 g sterilem Wasser bei 80°C gelöst. Die Lösung wird bei Raumtemperatur mit 1 N wässriger Natriumhydroxidlösung auf pH 7,4 gebracht. Danach werden 1,5 mg Synperonic® (PE/F 68, reinst Fa. ICI, CAS-Reg. No. 9003-11-6, Schmelzpunkt 77°C), 90,00 mg Mannit, 0,3 mg EDTA-Dinatriumsalz und 30,00 mg TPA-Wirkstoff (lyophilisiert) hinzugegeben. Man rührt, bis die Lösung klar wird und ergänzt mit 2,23 g sterilem Wasser. Die Lösung wird sterilfiltriert, in Vialen abgefüllt und lyophilisiert.

Beispiel 3: Injektionsdispersion enthaltend TPA

169,30 mg Sojalecithin (Sojaphosphatid NC 95, Fa. Nattermann, 90-96%-ig, Fettsäurezusammensetzung: Linolsäure 61-71 %, Oelsäure 6-13 %, Linolensäure 4-7 %, Palmitinsäure 10-15 %, Stearinsäure 1,5-3,5 %) und 92,70 mg Natriumglycocholat (reinst) werden in 752,5 mg sterilem Wasser bei 80°C gelöst. Die Lösung

wird bei Raumtemperatur mit 1 N wässriger Natriumhydroxidlösung auf pH 7.4 gebracht. Danach wird 10,00 mg Wirkstoff (lyophilisiert) hinzugegeben und gerührt, bis die Lösung klar wird. Die Lösung wird sterilfiltriert und in Ampullen abgefüllt.

Beispiel 4: Injektionsdispersion enthaltend TPA

169,30 mg Sojalecithin (Sojaphosphatid NC 95, Fa. Nattermann, 90-96%-ig, Fettsäurezusammensetzung: Linolsäure 61-71 %, Oelsäure 6-13 %, Linolensäure 4-7 %, Palmitinsäure 10-15 %, Stearinsäure 1,5-3,5 %) und 92,70 mg Natriumglycocholat (reinst) werden in 712,0 mg sterilem Wasser bei 80° C gelöst. Die Lösung wird bei Raumtemperatur mit 1 N wässriger Natriumhydroxidlösung auf pH 7,4 gebracht. Danach werden 1,00 mg Synperonic® (PE/F 68, reinst Fa. ICI, CAS-Reg. No. 9003-11-6, Schmelzpunkt 77° C), 30,00 mg Mannit und 20,00 mg TPA-Wirkstoff hinzugegeben. Man rührt, bis die Lösung klar wird. Die Lösung wird sterilfiltriert und in Ampullen abgefüllt.

Beispiel 5: Injektionsdispersion enthaltend TPA

338,60 mg Sojalecithin (Sojaphosphatid NC 95, Fa.Nattermann, 90-96%-ig, Fettsäurezusammensetzung: Linolsäure 61-71 %, Oelsäure 6-13 %, Linolensäure 4-7 %, Palmitinsäure 10-15 %, Stearinsäure 1,5-3,5 %) wird in 150 mg Ethanol gelöst und darin 185,40 mg Glycocholsäure (reinst) suspendiert. Die Lösung wird bei Raumtemperatur mit 1,013 ml 1,5%-iger (G/V) wässriger Natriumhydroxidlösung versetzt. Man rührt, bis man eine klare Lösung erhält und bringt durch Zugabe von 1 N Salzsäure auf pH 7.4. Dazu gibt man 1,5 mg Synperonic® (PE/F 68, reinst Fa. ICI, CAS-Reg. No. 9003-11-6, Schmelzpunkt 77° C) und 30,00 mg Wirkstoff (lyophilisiert) hinzu. Man rührt wiederum bis man eine klare Dispersion erhält und füllt mit Wasser auf 2,0 ml auf. Die Dispersion wird sterilfiltriert und in Ampullen abgefüllt.

Beispiel 6: Trockenpräparat enthaltend TPA

338,60 mg Sojalecithin (Sojaphosphatid NC 95, Fa. Nattermann, 90-96%-ig, Fettsäurezusammensetzung: Linolsäure 61-71 %, Oelsäure 6-13 %, Linolensäure 4-7 %, Palmitinsäure 10-15 %, Stearinsäure 1,5-3,5 %) wird in 150 mg Ethanol gelöst und darin 185,40 mg Glycocholsäure suspendiert. Die Lösung wird bei Raumtemperatur mit 1,013 ml 1,5%-iger (G/V) wässriger Natriumhydroxidlösung versetzt. Man rührt, bis man eine klare Lösung erhält und bringt durch Zugabe von 1 N Salzsäure auf pH 7.4. Dazu gibt man 60,00 mg Mannit (reinst) und 30,00 mg TPA-Wirkstoff (lyophilisiert) hinzu. Man rührt wiederum, bis man eine klare Lösung erhält und füllt mit Wasser auf 2,0 ml auf. Die Lösung wird sterilfiltriert, in Vialen abgefüllt und lyophilisiert.

Beispiel 7: Trockenpräparat enthaltend einkettige, hochmolekulare Urokinase

338,60 mg Sojalecithin (Sojaphosphatid NC 95, Fa. Nattermann, 90-96%-ig, Fettsäurezusammensetzung: Linolsäure 61-71 %, Oelsäure 6-13 %, Linolensäure 4-7 %, Palmitinsäure 10-15 %, Stearinsäure 1,5-3,5 %) wird in 150 mg Ethanol gelöst und darin 185,40 mg Glycocholsäure suspendiert. Die Lösung wird bei Raumtemperatur mit 1,013 ml 1,5%-iger (G/V) wässriger Natriumhydroxidlösung versetzt. Man rührt, bis man eine klare Lösung erhält und bringt durch Zugabe von 1 N Salzsäure auf pH 7.4. Dazu gibt man 60,00 mg Mannit (reinst) und 30,00 mg einkettige, hochmolekulare Urokinase (lyophilisiert), deren Herstellung im Journal of Biological Chemistry, 261 1274-1278 (1986) beschrieben ist, hinzu. Man rührt wiederum, bis man eine klare Lösung erhält und füllt mit Wasser auf 2,0 ml auf. Die Lösung wird sterilfiltriert, in Vialen abgefüllt und lyophilisiert.

**Patentansprüche**

1. Pharmazeutische Zusammensetzungen enthaltend

a) ein Phospholipid der Formel

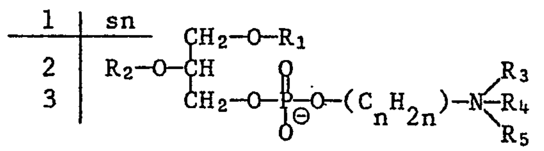

(I),

worin n zwei, drei oder vier ist, $R_1$ und $R_2$ unabhängig voneinander Alkyl, Alkenyl oder Acyl mit je 10-20 C-Atomen und $R_3$, $R_4$ und $R_5$ Wasserstoff oder $C_1$-$C_4$-Alkyl bedeuten,

b) ein Gallensäurederivat der Formel

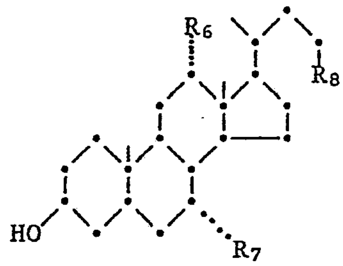

(II),

worin $R_6$ und $R_7$ unabhängig voneinander Wasserstoff, Hydroxy oder Oxo und $R_8$ Carboxy, verestertes oder amidiertes Carboxy bedeuten, und welches im Steroidgerüst ein oder zwei Doppelbindungen enthalten kann,

c) ein hochmolekulares Protein mit fibrinolytischer Aktivität (Fibrinolytikum) und gegebenenfalls eine pharmazeutisch annehmbare Trägerflüssigkeit und/oder für die pharmazeutische Formulierung verwendbare Hilfsstoffe.

2. Pharmazeutische Zusammensetzungen gemäss Anspruch 1, enthaltend

a) ein synthetisches oder natürliches Phospholipid der Formel I, worin n zwei ist, $R_1$ und $R_2$ Acyl mit je 10-20 C-Atomen und $R_3$, $R_4$ und $R_5$ Methyl bedeuten,

b) ein Gallensäurederivat der Formel II, worin $R_6$ Hydroxy, $R_7$ Wasserstoff oder Hydroxy und $R_8$ Carboxy, Sulfo oder 2-Carboxy- oder 2-Sulfoethylaminocarbonyl bedeuten,

c) ein hochmolekulares Protein mit fibrinolytischer Aktivität und gegebenenfalls eine pharmazeutisch annehmbare Trägerflüssigkeit und/oder für die pharmazeutische Formulierung verwendbare Hilfsstoffe.

3. Pharmazeutische Zusammensetzungen gemäss Anspruch 1 enthaltend

a) ein Phospholipid der Formel I, worin n zwei ist, $R_1$ und $R_2$ verschiedene oder identische $C_{10}$ -$C_{20}$ -Alkanoyl- oder $C_{10}$ -$C_{20}$ -Alkenoylgruppen und $R_3$, $R_4$ und $R_5$ Methyl bedeuten,

b) ein Gallensäurederivat der Formel II, worin $R_6$ und $R_7$ Hydroxy und $R_8$ 2-Carboxy- oder 2-Sulfoethylaminocarbonyl bedeuten,

c) einkettige Urokinase, welches aus menschlichem Urin oder dem Kulturfiltrat von Nierenzellkulturen oder genetisch rekombinierten Mikroorganismen oder Säugerzellen erhältlich ist, TPA aus Zellkulturen von gesundem oder tumorösem Zellgewebe von Säugetieren oder Menschen, TPA exprimiert in Kulturen von genetisch rekombinierten Mikroorganismen, z.B. Eukaryoten wie Pilzen, z.B. Aspergillusarten, oder Hefen, oder Prokaryoten, z.B. Escherichia Coli, oder genetisch rekombinierten Säugerzellen, Mutanten und Hybridformen von Urokinase und TPA, Konjugate von TPA, z.B. mit Proteinen, sowie denaturiertes TPA mit fibrinolytischer Wirkung oder durch Ersatz von Aminosäurefragmenten bzw. Derivatisierung am C- und/oder N-Terminus chemisch modifiziertes TPA und gegebenenfalls eine pharmazeutisch annehmbare Trägerflüssigkeit und/oder für die pharmazeutische Formulierung verwendbare Hifsstoffe.

4. Pharmazeutische Zusammensetzungen gemäss Anspruch 1 enthaltend

a) ein Phospholipid der Formel I natürlicher Herkunft aus Pflanzen oder Tieren, worin $R_1$ und $R_2$ unabhängig voneinander n-Dodecanoyl, n-Tetradecanoyl, n-Hexadecanoyl, n-Octadecanyol, 9-cis-Dodecenoyl, -Tetradecenoyl oder -Hexadecenoyl oder 6-cis, 6-trans-, 9-cis-, 9-trans- oder 11-cis-Octadecenoyl und $R_3$, $R_4$ und $R_5$ Methyl darstellen,

b) ein Gallensäurederivat der Formel II, worin $R_6$ und $R_7$ Hydroxy und $R_8$ 2-Carboxy- oder 2-Sulfoethylaminocarbonyl bedeuten,

c) einkettige Urokinase, welche aus menschlichem Urin oder dem Kulturfiltrat von Nierenzellkulturen oder genetisch rekombinierten Mikroorganismen oder Säugerzellen erhältlich ist, TPA aus Zellkulturen von gesundem oder tumorösem Zellgewebe von Säugetieren oder Menschen, TPA exprimiert in Kulturen von genetisch rekombinierten Mikroorganismen, z.B. Eukaryoten wie Pilzen, z.B. Aspergillusarten, oder Hefen, oder Prokaryoten, z.B. Escherichia Coli, und von genetisch rekombinierten Säugerzellen, oder Hybridformen von Urokinase und TPA und gegebenenfalls eine pharmazeutisch annehmbare Trägerflüssigkeit und/oder für die pharmazeutische Formulierung verwendbare Hilfsstoffe.

5. Verfahren zur Herstellung von pharmazeutischen Zusammensetzungen der Formel I, dadurch gekennzeichnet, dass man die Komponenten a), b) und c) und gegebenenfalls die Hilfsstoffe vermischt und in wässriger Phase dispergiert oder eine homogene Mischung aus den Komponenten und Hilfsstoffen bildet und diese in wässriger Phase dispergiert und, wenn notwendig, die wässrige Phase auf pH 7.2-7.4 abpuffert und gewünschtenfalls die erhältlichen Mischmizellen anreichert und/oder abtrennt.

6. Parenterales Verabreichungssytem auf der Basis von Mischmizellen für verkapseltes TPA, hergestellt nach dem Verfahren gemäss Anspruch 5.

7. Pharmazeutische Zusammensetzungen gemäss Anspruch 1 zur Anwendung bei der Behandlung des menschlichen oder tierischen Körpers.

8. Pharmazeutische Zusammensetzungen gemäss Anspruch 1 zur Anwendung bei der Prophylaze oder Therapie des menschlichen oder tierischen Körpers von Thrombosen.

9. Verwendung von Phospholipiden der Formel I, Gallensäurederivaten der Formel II, hochmolekularen Proteinen mit fibrinolytischer Aktivität und gegebenenfalls einer pharmazeutisch annehmbaren Trägerflüssigkeit und/oder für die pharmazeutische Formulierung verwendbarer Hilfsstoffe zur Herstellung von pharmazeutischen Präparaten für die Behandlung des menschlichen oder tierischen Körpers.

10. Verwendung gemäss Anspruch 9 zur Herstellung von pharmazeutischen Präparaten für die Prophylaxe oder Therapie des menschlichen oder tierischen Körpers von Thrombosen.

Patentansprüche für den folgenden Vertragsstaat: Oesterreich

1. Verfahren zur Herstellung von Pharmazeutischen Zusammensetzungen enthaltend

a) ein Phospholipid der Formel

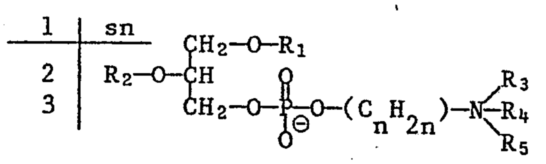

(I),

worin n zwei, drei oder vier ist, $R_1$ und $R_2$ unabhängig voneinander Alkyl, Alkenyl oder Acyl mit je 10-20 C-Atomen und $R_3$, $R_4$ und $R_5$ Wasserstoff oder $C_1$-$C_4$-Alkyl bedeuten,

b) ein Gallensäurederivat der Formel

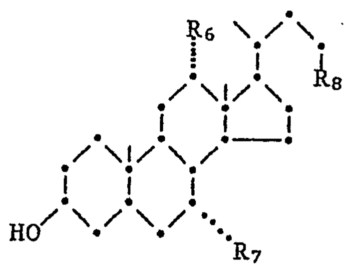

(II),

worin $R_6$ und $R_7$ unabhängig voneinander Wasserstoff, Hydroxy oder Oxo und $R_8$ Carboxy, verestertes oder amidiertes Carboxy bedeuten, und welches im Steroidgerüst ein oder zwei Doppelbindungen enthalten kann,

c) ein hochmolekulares Protein mit fibrinolytischer Aktivität (Fibrinolytikum) und gegebenenfalls eine pharmazeutisch annehmbare Trägerflüssigkeit und/oder für die pharmazeutische Formulierung verwendbare Hilfsstoffe, dadurch gekennzeichnet, dass man die Komponenten a), b) und c) und gegebenenfalls die Hilfsstoffe vermischt und in wässriger Phase dispergiert oder eine homogene Mischung aus den Komponenten und Hilfsstoffen bildet und diese in wässriger Phase dispergiert und, wenn notwendig, die wässrige Phase auf pH 7.2-7.4 abpuffert und gewünschtenfalls die erhältlichen Mischmizellen anreichert und/oder abtrennt.

2. Verfahren zur Herstellung von Pharmazeutischen Zusammensetzungen gemäss Anspruch 1 enthaltend

a) ein synthetisches oder natürliches Phospholipid der Formel I, worin n zwei ist, $R_1$ und $R_2$ Acyl mit je 10-20 C-Atomen und $R_3$, $R_4$ und $R_5$ Methyl bedeuten,

b) ein Gallensäurederivat der Formel II, worin $R_6$ Hydroxy, $R_7$ Wasserstoff oder Hydroxy und $R_8$ Carboxy, Sulfo oder 2-Carboxy- oder 2-Sulfoethylaminocarbonyl bedeuten,

c) ein hochmolekulares Protein mit fibrinolytischer Aktivität und gegebenenfalls eine pharmazeutisch annehmbare Trägerflüssigkeit und/oder für die pharmazeutische Formulierung verwendbare Hilfsstoffe, dadurch gekennzeichnet, dass man die in Anspruch 1 genannten Verfahrensmassnahmen ausführt.

3. Verfahren zur Herstellung von Pharmazeutischen Zusammensetzungen gemäss Anspruch 1 enthaltend

a) ein Phospholipid der Formel I, worin n zwei ist, $R_1$ und $R_2$ verschiedene oder identische $C_{10}$-$C_{20}$-Alkanoyl- oder $C_{10}$-$C_{20}$-Alkenoylgruppen und $R_3$, $R_4$ und $R_5$ Methyl bedeuten,

b) ein Gallensäurederivat der Formel II, worin $R_6$ und $R_7$ Hydroxy und $R_8$ 2-Carboxy- oder 2-Sulfoethylaminocarbonyl bedeuten,

c) einkettige Urokinase, welche aus menschlichem Urin oder dem Kulturfiltrat von Nierenzellkulturen oder genetisch rekombinierten Mikroorganismen oder Säugerzellen erhältlich ist, TPA aus Zellkulturen von gesundem oder tumorösem Zellgewebe von Säugetieren oder Menschen, TPA exprimiert in Kulturen von genetisch rekombinierten Mikroorganismen, z.B. Eukaryoten wie Pilzen,

z.B. Aspergillusarten, oder Hefen, oder Prokaryoten, z.B. Escherichia Coli, oder genetisch rekombinierten Säugerzellen, Mutanten und Hybridformen von Urokinase und TPA, Konjugate von TPA, z.B. mit Proteinen, sowie denaturiertes TPA mit fibrinolytischer Wirkung oder durch Ersatz von Aminosäurefragmenten bzw. Derivatisierung am C- und/oder N-Terminus chemisch modifiziertes TPA und gegebenenfalls eine pharmazeutisch annehmbare Trägerflüssigkeit und/oder für die pharmazeutische Formulierung verwendbare Hilfsstoffe, dadurch gekennzeichnet, dass man die in Anspruch 1 genannten Verfahrensmassnahmen ausführt.

4. Verfahren zur Herstellung von Pharmazeutischen Zusammensetzungen gemäss Anspruch 1 enthaltend

a) ein Phospholipid der Formel I natürlicher Herkunft aus Pflanzen oder Tieren, worin $R_1$ und $R_2$ unabhängig voneinander n-Dodecanoyl, n-Tetradecanoyl, n-Hexadecanoyl, n-Octadecanyol, 9-cis-Dodecenoyl, -Tetradecenoyl oder -Hexadecenoyl oder 6-cis, 6-trans-, 9-cis-, 9-trans- oder 11-cis-Octadecenoyl und $R_3$ , $R_4$ und $R_5$ Methyl darstellen,

b) ein Gallensäurederivat der Formel II, worin $R_6$ und $R_7$ Hydroxy und $R_8$ 2-Carboxy- oder 2-Sulfoethylaminocarbonyl bedeuten,

c) einkettige Urokinase, welche aus menschlichem Urin oder dem Kulturfiltrat von Nierenzellkulturen oder genetisch rekombinierten Mikroorganismen oder Säugerzellen erhältlich ist, TPA aus Zellkulturen von gesundem oder tumorösem Zellgewebe von Säugetieren oder Menschen, TPA exprimiert in Kulturen von genetisch rekombinierten Mikroorganismen, z.B. Eukaryoten wie Pilzen, z.B. Aspergillusarten, oder Hefen, oder Prokaryoten, z.B. Escherichia Coli, und von genetisch rekombinierten Säugerzellen, oder Hybridformen von Urokinase und TPA und gegebenenfalls eine pharmazeutisch annehmbare Trägerflüssigkeit und/oder für die pharmazeutische Formulierung verwendbare Hilfsstoffe, dadurch gekennzeichnet, dass man die in Anspruch 1 genannten Verfahrensmassnahmen ausführt.

Europäisches Patentamt

# EUROPÄISCHER RECHERCHENBERICHT

Nummer der Anmeldung

EP 87 81 0348

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 133 258 (F. HOFFMANN-LA ROCHE)<br>* Seite 1, Zeile 15 - Seite 3, Zeile 3; Ansprüche 1-8 *<br>--- | 1-10 | A 61 K 37/54<br>A 61 K 47/00 |
| Y | US-A- 158 707 (H. STEFFEN et al.)<br>* Spalte 1, Zeile 50 - Spalte 3, Zeile 29; Ansprüche 1-7 *<br>--- | 1-10 | |
| Y | EP-A-0 156 169 (ASAHI KASEI KOGYO K.K.)<br>* Seite 8, Zeile 11 - Seite 10, Zeile 18; Ansprüche 1-20 *<br>--- | 1-10 | |
| Y | EP-A-0 123 304 (ASAHI KASEI KOGYO K.K.)<br>* Seite 14, Zeilen 5-21; Ansprüche 1-24 *<br>----- | 1-10 | RECHERCHIERTE SACHGEBIETE (Int. Cl.4)<br>A 61 K |

Der vorliegende Recherchenbericht wurde für alle Patentansprüche erstellt.

| Recherchenort | Abschlußdatum der Recherche | Prüfer |
|---|---|---|
| DEN HAAG | 19-08-1987 | TZSCHOPPE,D.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument